# EUROPÄISCHE PATENTANMELDUNG

(11) **EP 2 491 965 A2**
(43) Veröffentlichungstag der Anmeldung: **29.08.2012**
(21) Anmeldenummer: 12154833.3
(22) Anmeldetag: 10.02.2012
(51) Int. Cl.: A61L 31/16, A61F 2/90

(54) **Implantat und Verfahren zur Herstellung desselben**

(30) Priorität: 24.02.2011 US 201161446049 P
(71) Anmelder: Biotronik AG, 8180 Bülach (CH)
(72) Erfinder: Diener, Tobias, 90763 Fürth (DE); Burean, Elisabeta, 91054 Erlangen (DE); Fringes, Matthias, 91522 Ansbach (DE)
(74) Vertreter: Lindner-Vogt, Karin L.

(57) **Zusammenfassung**

Die vorliegende Erfindung betrifft ein Implantat mit einer vorzugsweise hohlzylinderförmigen Grundstruktur mit einer Vielzahl von durchgehenden Öffnungen und mit einer Beschichtung, welche mindestens eine pharmazeutisch aktive Substanz freigibt. Um eine bessere Verteilung der pharmazeutisch aktiven Substanz zu erreichen, ist mindestens 20% der Querschnittsfläche, vorzugsweise mindestens 50% der Querschnittsfläche, mindestens eines Teils der Öffnungen eines vorgegebenen Abschnitts der Grundstruktur mit der mindestens eine pharmazeutisch aktive Substanz freigebenden Beschichtung bedeckt. Es werden ferner ein System aus einem Katheter und einem solchen Implantat, ein einfaches Verfahren zur Herstellung eines derartigen Implantats bzw. eines solchen Systems vorgeschlagen.

## Beschreibung

Die vorliegende Erfindung betrifft ein Implantat, insbesondere eine intraluminale Endoprothese, mit einer vorzugsweise hohlzylindrischen Grundstruktur, ein System aus einem Katheter und einem derartigen Implantat sowie ein Verfahren zur Herstellung eines solchen Implantats bzw. eines solchen Systems.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stent, Gallengang-Stent, Vascular Stent (einschließlich des Herzens und Herzklappenstents), Mitral-Stent), Endoprothesen zum Verschließen von persitierenden Foramen ovale (PFO), Pulmonalklappenstent (pulmonary valve stent), Endoprothesen zum Verschließen eines ASD (Vorhofscheidewanddefekt, atrial septal defect) sowie Prothesen im Bereich des Hart- und Weichgewebes zu verstehen.

Heutzutage werden als Implantate besonders häufig Stents eingesetzt, die zur Behandlung von Stenosen (Gefäßverengungen) dienen. Sie weisen eine durchbrochene rohrförmige oder hohlzylinderförmige Grundstruktur auf, die an beiden Längsenden offen ist. Die Grundstruktur besitzt somit eine Vielzahl von durchgehenden Öffnungen. Die Grundstruktur des Stents setzt sich dabei häufig aus einzelnen Maschen zusammen, welche aus verschieden geformten Stegen (Struts), beispielsweise zick-zack- oder mäander-förmigen Stegen, gebildet werden. Eine derartige Endoprothese wird häufig mittels eines Katheters in das zu behandelnde Gefäß eingesetzt und dient dazu, das Gefäß über einen längeren Zeitraum (Monate bis Jahre) zu stützen. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines Stents, der an sich einen Fremdkörper darstellt, eine Kaskade von mikrobiologischen Prozessen, die beispielsweise eine Entzündung (Inflammation) des behandelten Gefäßes oder eine nekrotische Gefäßveränderung begünstigen und/oder die zu einem allmählichen Zuwachsen des Stents durch Bildung von Plaques bzw. Koagulation von Körperflüssigkeit infolge einer Strömungsveränderung oder infolge eines Infektionsprozesses führen können.

Um Restenosen bzw. Inflammationen und Nekrosen zu vermeiden, werden Stents oder andere Implantate häufig mit einer Beschichtung von Medikamenten versehen, die beispielsweise eine antikoagulierende oder eine antünflammatorische Wirkung besitzen. Hierbei ist es wünschenswert, dass die Implantate die pharmazeutisch aktiven Substanzen gezielter freigeben.

Es sind bereits Stents mit Medikamentenabgabe aus einer Beschichtung bekannt, die beispielsweise aus einem Polymer bestehen kann. Außerdem werden bereits Medikamentendepots mit Pumpen verwendet. Nachteilig an den bekannten Lösungen ist jedoch, dass die pharmazeutisch aktiven Substanzen permanent abgegeben werden und keine Regelung der Freisetzung möglich ist. Zudem ist lediglich eine konstante Dosierung der Substanzen realisierbar und der Restbestand des Medikaments ist im Reservoir oder Depot nicht feststellbar. Der Organismus des behandelten Menschen oder Tieres wird durch die Dauerabgabe der Inhaltsstoffe unnötig belastet.

Aus der Druckschrift US 7,060,093 B2 ist ein Stent bekannt, welcher Ausnehmungen (Depots) in den Stegen aufweist. Diese Depots enthalten Medikamente und geben diese später ab. Die Medikamentenabgabe aus solchen Depots ist ebenfalls schlecht kontrollierbar und weist eine bezogen auf die Innenoberfläche des behandelten Gefäßes ungünstige Verteilung der Medikamente über diese Fläche auf. Dies führt ebenfalls zu einer größeren Belastung des behandelten Organismus, da, um eine ausreichende Medikamentenversorgung in allen notwendigen Bereichen des behandelten Organs sicherzustellen, lokal eine Überdosierung bewirkt wird, welche toxische Reaktionen hervorrufen kann.

Die Aufgabe der vorliegenden Erfindung besteht somit darin, ein Implantat zu schaffen, das eine gezieltere und den Organismus weniger belastende Abgabe eines Medikaments ermöglicht. Außerdem soll ein kostengünstiges System aus einem Implantat und eine Katheter geschaffen sowie ein einfaches Verfahren für die Herstellung eines derartigen Implantats bzw. eines solchen Systems angegeben werden.

Die obige Aufgabe wird durch ein Implantat gelöst, bei dem mindestens 20% der Querschnittsfläche, vorzugsweise mindestens 50% der Querschnittsfläche, mindestens eines Teils der Öffnungen eines vorgegebenen Abschnitts der Grundstruktur, vorzugsweise der Öffnungen des vorgegebenen Abschnitts mit der kleinsten Querschnittsfläche, besonders bevorzugt höchstens 95% der Summe der Querschnittsflächen aller Öffnungen des vorgegebenen Abschnitts, im dilatierten Zustand mit der mindestens eine pharmazeutisch aktive Substanz freigebenden Beschichtung bedeckt bzw. überspannt ist. Hierfür enthält die Beschichtung die mindestens eine pharmazeutisch aktive Substanz beispielsweise in Poren. Hierbei können im nicht dilatierten Zustand, beispielsweise im gecrimpten Zustand, in dem das Implantat auf einen Ballon aufgecrimpt ist, die eine Beschichtung aufweisenden Öffnungen vollständig bedeckt sein. Erst während des Dilatierens kann die Beschichtung z.B. entlang einer Sollbruchstelle einreißen, so dass im expandierten Zustand ein geringerer Teil der Querschnittsfläche der jeweiligen Öffnungen bedeckt ist. Hierbei kann eine Berechnung zum Verhalten der Beschichtung in einer Simulation über eine FEM-Analyse (Finite-Elemente-Analyse) vorab erfolgen. Hierdurch können gezielt die Eigenschaften der Beschichtung eingestellt werden.

In Bezug auf die vorliegende Erfindung ist mit der Bezeichnung 'Querschnittsfläche' die Fläche gemeint, welche die jeweilige Öffnung entlang der abluminalen Ebene des Mantels des Implantats aufspannt. Anders ausgedrückt entspricht die Querschnittsfläche der Fläche der Öffnung, die sichtbar ist, wenn ein Betrachter von außen auf die Öffnung blickt. Die Beschichtung erstreckt sich von der die jeweilige Öffnung umgebenden Grundstruktur in die Öffnung hinein und überspannt diese ähnlich einer 'Haut'. Vorzugsweise beträgt die Schichtdicke der Beschichtung maximal 100 µm.

Das erfindungsgemäße Implantat hat den Vorteil, dass nun eine große Fläche für die Abgabe von Medikamenten zur Verfügung steht. Diese Fläche ist beispielsweise deutlich größer als die Fläche der Stege, die im Stand der Technik zur Abgabe des Medikaments dienen. Eine gleichmäßigere Verteilung der pharmazeutisch aktiven Substanz über die zu behandelnde Fläche, beispielsweise die Innenwand eines Gefäßes, wird somit erreicht. Folglich kann eine Überdosierung der pharmazeutisch aktiven Substanz im Bereich des behandelten Organs vermieden werden, so dass Nebeneffekte, z.B. toxische Reaktionen, nicht mehr auftreten.

Aufgrund der großen, für die Abgabe der pharmazeutisch aktiven Substanz zur Verfügung stehenden Fläche ist zudem eine luminale Abgabe der pharmazeutisch aktiven Substanz nicht notwendig, die abluminale Versorgung mit der pharmazeutisch aktiven Substanz ist ausreichend. Folglich ist es erfindungsgemäß vorgesehen, dass die pharmazeutisch aktive Substanz mindestens überwiegend in abluminaler Richtung aus der Beschichtung abgegeben wird. Vorzugsweise wird mindestens 95 Gew.% der mindestens einen pharmazeutisch aktiven Substanz aus der Beschichtung in abluminaler Richtung abgegeben. Dies ist deshalb von Vorteil, weil die luminale Abgabe von Medikamenten ein Einwachsen des Implantats bzw. die Endothelialisierung behindern kann. Bei der erfindungsgemäßen Lösung ist daher aufgrund der fehlenden luminalen Abgabe des Medikaments eine ausreichende Endothealialisierung des Implantats gegeben und es kann eine Spätthrombose vermieden werden. Die Abgabe der mindestens einen pharmazeutisch aktiven Substanz erfolgt durch Aufdehnung von Poren, welche die Beschichtung aufweist, während und nach der Dilatation des Implantats. Die Freisetzung der in der Matrix der Beschichtung angeordneten mindestens einen pharmazeutisch aktiven Substanz wird somit durch die Dilatation des Implantats erst initialisiert bzw. angeregt. Weiterhin bieten die geöffneten Poren der Beschichtung eine Angriffsfläche für die Degradation der vorzugsweise aus einem Polymer bestehenden Matrix der Verwendung, wenn degradierbare Materialien für die Beschichtungsmatrix eingesetzt werden.

Die Poren für die Aufnahme der pharmazeutisch aktiven Substanz können beispielsweise durch Verwendung eines porösen Polymers als Beschichtungsmaterial bereitgestellt werden. Alternativ oder zusätzlich können Poren auf mechanischem Wege, z.B. durch Stanzen, erzeugt werden. Hierbei wird das Material der Beschichtung zunächst stark gedehnt, dann werden die Poren eingestanzt und danach wird das Material wieder entlastet. Das Befüllen erfolgt dann ebenfalls unter Dehnung des Beschichtungsmaterials. Nach der Aushärtung der Füllung, d.h. der pharmazeutisch aktiven Substanz, wird das Beschichtungsmaterial entlastest, so dass das Polymer in seine Ausgangsform zurückgeht. Bei den oben beschriebenen Dehnungen des Beschichtungsmaterials ist der Dehnungsbereich der plastischen Verformung zu meiden. Dieses Verfahren gilt sowohl für "normale" Polymere als auch für Formgedächtnis-Polymere. Eine weitere Möglichkeit, Poren in die Beschichtung einzubringen, ist das Ätzen.

Ferner ist es möglich, das Implantat für eine überwiegend abluminale Abgabe der pharmazeutisch aktiven Substanz zu präparieren. Nach einem Ausführungsbeispiel der vorliegenden Erfindung weist die Beschichtung mehrere Lagen (Schichten) auf, wobei die erste, am weitesten in luminaler Richtung angeordnete Lage vorzugsweise als Diffusionsbarriere für die mindestens eine pharmazeutisch aktive Substanz ausgebildet ist. Folglich wird bei diesem Ausführungsbeispiel eine mehrschichtige Beschichtung verwendet, wobei zuerst eine Lage (Schicht) aufgetragen wird, welche keine pharmazeutisch aktive Substanz enthält. Diese vorzugsweise aus einem Polymer bzw. einem degradierbaren Material bestehende Lage dient als Diffusionsbarriere oder Diffusionssperre für die pharmazeutisch aktive Substanz und ist in der Beschichtung am weitesten in luminaler Richtung angeordnet. Nachdem diese Schicht ausgehärtet ist, wird darüber mindestens eine weitere Schicht aufgetragen, welche in ihrer Matrix eingebettet mindestens eine pharmazeutisch aktive Substanz aufweist. Das Material der Matrix kann auch biodegradierbar ausgeführt sein.

In einem weiteren Ausführungsbeispiel können in der Beschichtung eine oder mehrere Sollbruchstellen (z.B. als Sollbruchlinie in Form von entlang einer Linie angebrachten Lochperforationen in der Beschichtung oder einer linienförmigen Verringerung der Schichtdicke der Beschichtung) vorgesehen sein, welche während der Dilatation des Implantats definiert reißen. Dies begünstigt insbesondere die Freisetzung der in der Beschichtung enthaltenen pharmazeutisch aktiven Substanz.

Erfindungsgemäß ist vorgesehen, ggf. nur vorgegebene Abschnitte der Grundstruktur, beispielsweise nur die Enden des Implantats in Richtung einer Längsachse des Implantats mit der Beschichtung mit der pharmazeutisch aktiven Substanz zu versehen, um Effekte wie Dogboning, strömungsinduzierte Proliferation an den Enden des Implantats oder dergleichen zu verhindern. Das in dem vorgegebenen Abschnitt der Grundstruktur nur lokal verabreichte Medikament kann gezielt dort wirken, wo es benötigt wird, und muss nicht in unnötiger Weise entlang der gesamten Achse den Implantats angeordnet werden.

Aufgrund der insgesamt notwendigen geringeren Konzentration der pharmazeutisch aktiven Substanz wird auch die Degradation des Implantats, falls die Grundstruktur aus einem degradierbaren Material besteht, nicht unnötig durch die pharmazeutisch aktive Substanz beeinflusst. Die Substanz kann so ungestört eluiert werden und effektiv gegen eine Restenose wirken.

Im Rahmen der vorliegenden Erfindung wird unter einer pharmazeutisch aktiven Substanz (oder therapeutisch aktive oder wirksame Substanz) ein pflanzlicher, tierischer oder synthetischer Wirkstoff (Medikament) oder ein Hormon verstanden, das in geeigneter Dosierung als Therapeutikum zur Beeinflussung von Zuständen oder Funktionen des Körpers, als Ersatz für natürlich vom menschlichen oder tierischen Körper erzeugte Wirkstoffe, wie Insulin, sowie zur Beseitigung oder zum Unschädlichmachen von Krankheitserregern, Tumoren, Krebszellen oder Körperfremdstoffen Einsatz findet. Die Freisetzung der Substanz in der Endoprothesenumgebung hat einen positiven Effekt auf den Heilungsverlauf oder wirkt pathologischen Veränderungen des Gewebes in Folge des chirurgischen Eingriffs entgegen bzw. dient dem Unschädlichmachen von maladen Zellen in der Onkologie.

Derartige pharmazeutisch aktive Substanzen weisen beispielsweise eine antiinflammatorische und/oder antiproliferative und/oder spasmolytische Wirkung auf, wodurch beispielsweise Restenosen, Entzündungen oder (Gefäß-)Spasmen vermieden werden können. Derartige Substanzen können in besonders bevorzugten Ausführungsbeispielen aus einer oder mehreren Substanzen der Wirkstoffgruppe der Calciumkanalblocker, der Lipidregulatoren (wie beispielsweise Fibrate), der Immunsuppressiva, der Calcineurininhibitoren (wie beispielsweise Tacrolimus), der Antiflogistika (wie beispielsweise Cortison oder Dichlofenac), der Antiinflammatorica (wie beispielsweise Imidazole), der Antiallergika, der Oligonucleotide (wie beispielsweise dODN), der Östrogene (wie beispielsweise Genistein), der Endothelbildner (wie beispielsweise Fibrin), der Steroide, der Analgetika, der Antirheumatika der Proteine, der Hormone, der Insuline, der Zytostatika, der Peptide, der Vasodilatatoren (wie beispielsweise Sartane) und der antiproliferativ (proliferationshemmend) wirkenden Stoffe der Taxole oder Taxane, hier vorzugsweise Paclitaxel oder Sirolimus, bestehen, oder können der folgenden Liste entnommen werden: Cisplatin, Tirapazamine, Enzyme L-asparaginase, Methotrexate, 5-Fluorouracil, Azathioprine, Mitoxantrone, Cyclophosphamide, Methotrexate, Natalizumab, Adriamycin PFS, Adriamycin RDF, Alitretinoin, Altretamine, Aromasin, Azathioprine, Bicalutamide, Busulfan, Busulfex, Capecitabine, Casodex, Cyclophosphamide, Cytoxan, Doxorubicin, Exemestane, Femara, Finasteride, Gemtuzumab, Ozogamicin, Hexalen, Imuran, Letrozole, Mifeprex, Mifepristone, Myleran, Mylotarg, Neosar, Nolvadex, Panretin, Propecia, Proscar Rubex, Tamoxifen, Temodar, Temozolomide, Trelstar Depot, Triptorelin, Genasense (Fa.Genta), INGN201 (Fa.Introgen Therapeutics), SCH58500 (Fa.Schering-Plough), ONYX-015 (Fa.Onyx Pharmaceuticals), E1A-lipid complex (Fa.Targeted Genetics), TRAIL (Fa.Genentech/Immunex), GX01 (Fa.Gemin X Biotechnologies), Cyclosporin A, DPPE, PSC 833, Buthionine sulphoximine, Dexverapamil, Quinine, Verapamil, XR9576, Dexniguldipin, GF120918, Lobradimil, LY335979, MS209, R-101933, Gemtuzumabozogamicin, SGN-15, MCC-465, SB-408075, A5B7 antibody against CEA with carboxy peptidase A + mustard prodrug, Amifostine, Dexrazoxane, BB-10010, Transfer of MDR genes, BNP7787, Tirapazamine, Aplidine, Arsenic trioxide, BMS-247550, CHS828, CT 2584, Dolastatin-10, ET-743, Exisulind, Irofulven, KW-2189, Lovastatin, E7070, LU103793, LY355703, Pyrazoloacridine, TLK286, Apomine, CP-461, EP0906, FB642, FK317, FK866, Kahalalide F, LAF389, PNU-166196, RO 31-7453, Cetuximab (Erbitux), Trastuzumab (Herceptin), ABX-EGF, AP12009, EMD55900, EMD72000, ICR62, 2A11, CCI-779, ISIS-3521, Oblimersen (Genasense), OSI-774 (Tarceva), PS-341, R115777 (Zarnestra), STI571 (Gleevec), ZD1839 (Iressa), Bryostatin-1, Flavopiridol, GD0039, GEM231, Ilmofosine, ISIS-2503, ISIS-5132, L-778 123, PKC 412, SCH66336, SU-101, UCN-01, Bay 43-9006, BMS-214662, CI-1040, GW572016, LErafAON, LY-317615, Perifosine, Phenoxodiol, PKI 166, Swainsonine, 17-AAG, Decitabine, CI-994, Depsipeptide, MG98, Phenylbutyrate, Phenylacetate, Suberoylanilidehydroxamic acid, Adp53, Antineoplastons, A10/AS2-1, OL(1)p53, p53, RPR/INGN-201, SCH 58500, HSV-TK VPC, tgDCC-E1A, INX3280, TK gene Pioglitazone, Troglitazone, BAY12-9566, BMS-275291, Clodronate, Marimastat, Prinomastat, MMI270, COL-3, CP-471,358, trans retinoic acid, Bexarotene, Pivaloyloxymethylbutyrate, 9-cis-retinoic acid, 13-cis RA, Fenretinide, ILX23-7553, TAC-101, Tazarotene, Bevacizumab, RhuMab-VEGF, HuMV833, Angiozyme, IMC-1C11, PI-88, SU5416, CP547,632, PNU-145156E, PTK/ZK 787, SU6668, ZD6474, Carboxyamidotriazole, GBC-590, Squalamine, Vitxain, ABT-510, CM101, ZD6126, Neovastat, Suramin, Thalidomide, IM862, TNP-470, Angiostatin, CC-5013, Combretastatin A4, Endostatin, Interleukin-12, Alemtuzumab, Edrecolomab, Epratuzumab HuM195, Oregovomab, Rituximab, Ch14.18, MDX-11, WX-G250, 3F8, H22xKI-4, ING-1, J591, KM871, Immunoconjugates antibody with toxin, BL22, Anti-Tac-PE38 (LMB-2), BB-10901, SS1-PE38, Denileukin diftitox (ONTAK), IL13-PE38QQR, TP-38, Allovectin-7, 105AD7, BEC2, TriGem, 1A7, 3H1, Vaccines, MDX-H210, G17DT, MDX-447, EMD 273063, IL-2/histamine, LAK, TIL, CTL, Bay 50-4798, MDX-010, OK-432, PSK, Ubenimex, GM-CSF, ONYX-015, NV1020, PV701, Reolysin, Celecoxib, Lyprinol, LY293111, Astrasentan, Melatonin, Taurolidine, Cyclosporin A, Verapamil, Tirapazamine Trastuzumab, Clodronate, trans retinoic acid, Edrecolomab, Rituximab, OK-432, Ubenimex, Melatonin, PSC 833, R115777, ZD1839, SCH 66336, Decitabine, HSV-TK, VPC, BAY12-9566, Marimastat, Prinomastat, Suramin, 105AD7, IL-2/histamine, Astrasentan.

Um eine genügend große Ausdehnung der Beschichtung zu erreichen, ist es häufig ausreichend, wenn höchstens jede zweite der nebeneinander liegenden, durchgehenden Öffnungen des vorgegebenen Abschnitts der Grundstruktur mit der mindestens eine pharmazeutisch aktive Substanz freigebenden Beschichtung bedeckt bzw. überspannt ist.

Ein besonders einfacher Aufbau des Implantats, insbesondere für Stents, wird dadurch erreicht, dass die Grundstruktur aus abgeschlossenen Zellen oder Maschen zusammengesetzt ist, wobei jede Zelle eine durchgehende Öffnung aufweist. Vorzugsweise wird jede Zelle durch zwei oder mehr, gegebenenfalls geknickte und/oder gebogene Stege gebildet.

Es ist von Vorteil, wenn das Material der Beschichtung, welche die pharmazeutisch aktive Substanz enthält, elastisch ausgebildet ist. Vorzugsweise weist das Material der Beschichtung einen E-Modul im Bereich von 0,01 kN/mm² bis 4 kN/mm², vorzugsweise im Bereich von 0,01 kN/mm² bis 2 kN/mm², auf. Das entsprechende Dehnvermögen bzw. die entsprechende Elastizität des Materials der Beschichtung beträgt 100% bis 800%, vorzugsweise 300% bis 800%. Hierdurch ist es möglich, eine Dilatation des Implantats durchzuführen, ohne dass die Beschichtung beschädigt wird.

Ferner ist von Vorteil, wenn aufgrund einer beispielsweise dilatationsbedingten Dehnung des Materials der Beschichtung die Elution (Abgabe) der pharmazeutisch aktiven Substanz in bestimmten Bereichen der Beschichtung, beispielsweise in der Mitte einer Beschichtung einer Öffnung, schneller beginnt als in anderen Bereichen. Hierdurch ist gewährleistet, dass die pharmazeutisch aktive Substanz über einen längeren Zeitraum freigegeben wird und nicht sofort zu Beginn der Behandlung komplett ausgespült wird. Diese Kinetik lässt sich anhand der durch die Mechanik der Grundstruktur gegebenen Dilatation und des Designs der Beschichtung steuern.

Es ist weiter bevorzugt, wenn die Beschichtung mindestens ein Polymer der Gruppe enthaltend die Polymere der Klasse der Elastomere, insbesondere Kautschuk und Latex, weiter enthaltend PET (Polyethylenterephthalat), PS (Polystyrol), PA (Polyamid), PUR (Polyurethan), PE (Polyethylen), PES (Polyethersulfon), PTFE (Polytetrafluorethylen, Teflon®), PLA (Polylactid, Polymilchsäuren), PLLA (Poly-L-Lactid), PLGA (Poly-L-Glycolide), PGA (Polyglycolide), PBMA (Poly-Butyl-Methacrylate), PEVA (Polyethylen-Vinylacetat), Formgedächtnispolymere, sowie deren Blends sowie Co- und Blockpolymere. Beispiele für Polymere mit Formgedächtniseffekt sind Multiblock-Copolymere oder Standardpolymere (PET, PS, PUR, PE, PES, PTFE). Die genannten Polymere sind besonders gut dazu geeignet, eine pharmazeutisch aktive Substanz in verschiedenster Form aufzunehmen und diese am Behandlungsort auch wieder abzugeben, da diese Polymere hoch elastisch sind und bei kleinen Dehnungen nicht reißen. Ferner haben viele von den genannten Polymeren die oben angegebenen Eigenschaften in Bezug auf ihre Dehnbarkeit, da das Implantat häufig am Behandlungsort dilatiert wird. Bevorzugt ist das Material der Beschichtung biokompatibel und ggf. auch degradierbar.

Formgedächtnis-Polymere (FGP) sind Kunststoffe, die einen Shape-Memory-Effekt aufweisen, also sich an ihre frühere äußere Form trotz einer zwischenzeitlichen starken Umformung scheinbar "erinnern" können. Derartige Formgedächtnis-Polymere bestehen in der Regel aus zwei Komponenten. Die erste ist ein elastisches Polymer, eine Art "Federelement". Die zweite Komponente ist ein aushärtendes Wachs, welches das "Federelement" nach seiner Aushärtung in jeder gewünschten Form arretieren kann. Wird nun das Formgedächtnis-Polymer erwärmt, so wird das Wachs weich und kann deshalb der Kraft des elastischen Polymers (1. Komponente) nicht mehr entgegenwirken. Das Formgedächtnis-Polymer nimmt in diesem Zustand wieder seine (ursprüngliche) Form an. Die oben genannten Formgedächtnis-Polymere (FGP) sind besonders gut dazu geeignet, eine pharmazeutisch aktive Substanz in verschiedenster Form aufzunehmen und diese am Behandlungsort auch wieder abzugeben, da in diese Polymere Poren "einprogrammiert" werden können, die erst während und nach der Dilatation am Implantationsort geöffnet werden und somit die mindestens eine pharmazeutisch aktive Substanz freigeben können. Die Bereitstellung oder Herstellung ("Einprogrammierung") von Poren in dem Beschichtungsmaterial enthaltend mindestens ein Formgedächtnis-Polymer wurde bereits oben dargestellt.

In einem weiteren Ausführungsbeispiel wird die Beschichtung, welche schicht- oder hautförmig gestaltet ist, mittels einer Klebeverbindung, einem Formschluss und/oder einem Stoffschluss (d.h. mittels einer chemischen Verbindung) mit der Grundstruktur verbunden, beispielsweise ist die Beschichtung mit der pharmazeutisch aktiven Substanz an der die Grundstruktur ausbildenden Stegen befestigt. Hierzu weist die Grundstruktur des Implantats, vorzugsweise ein Teil der Stege, in einem Ausführungsbeispiel vorzugsweise auf der abluminalen Seite eine Nut zur Befestigung der Beschichtung auf

Die Grundstruktur eines derartigen Implantats kann vorzugsweise mindestens ein Element und/oder eine Verbindung aus der folgenden Gruppe enthaltend Metalle, Metalllegierungen, vorzugsweise Edelstahl, CoCr, Magnesiumlegierungen, Eisenlegierungen, Zinklegierungen, Manganlegierungen, Nitinol, Polymere aus der Klasse der bioabbaubaren Polymere, vorzugsweise Polymilchsäuren, Poly-caprolactone, Mischungen oder Copolymere daraus, Polymere aus der Klasse der biokompatiblen Polymeren, vorzugsweise UHMWPE und PEEK, enthalten. Hierbei wird ein Implantat gestaltet als Stent bestehend aus einer biodegradierbaren Magnesiumlegierung, Eisenlegierungen, Zinklegierung oder Manganlegierung als AMS (= absorbierbarer Metallstent) bezeichnet.

Die Ermittlung der Position eines Stents geschieht häufig mittels bildgebender Verfahren, beispielsweise mittels einer Röntgenstrahleinrichtung. Da die für das Grundgitter von Stents verwendeten Materialien in der Regel Röntgenstrahlung nur in geringem Maße absorbieren, d.h. röntgen- oder radioluzent sind, wird das Implantat in einem Ausführungsbeispiel der Erfindung mit mindestens einem Röntgenmarker versehen, welcher ein Material enthält, das eine höhere Absorption der Röntgenstrahlen aufweist (auch als röntgenopakes oder radioopakes Material bezeichnet) als das ihn umgebende Material. Das röntgenopake Material ist in der Beschichtung vorzugsweise in flüssiger Form, z.B. als reines Jod, enthalten. Hierbei wird das flüssige röntgenopake Material während des Beschichtungsprozesses zusammen mit dem Material der Matrix verarbeitet. Es härtet später aus oder wird auf andere Weise fest und liegt nach dem Abschluss des Beschichtungsprozesses, d.h. in dem aufgecrimpten Stent bzw. dem in den Körper eingebrachten und gegebenenfalls dilatierten Stent, in fester Form vor. Alternativ kann das röntgenopake Material in gekapselter Form vorliegen. Hierbei ist das Material der Mikrokapsel biokompatibel und nicht resorbierbar. Das röntgenopake Material kann beispielsweise wie Gold-, Silber- oder Tantalpartikel umfassen. Die Mikrokapseln haben vorzugsweise eine Größe von 0,5 µm bis 1 µm.

Ein solcher Röntgenmarker weist röntgenopakes Material auf, nämlich eines oder mehrere der Elemente und/oder Verbindungen aus der Gruppe enthaltend Gold, Platin, Silber, Wolfram, Iod, Tantal, Yttrium, Niob, Molybdän, Ruthenium, Rhodium, Barium, Lanthan, Cer, Praseodym, Neodym, Samarium, Europium, Gadolinium, Terbium, Dysprosium, Holmium, Erbium, Thulium, Ytterbium, Lutetium, Hafnium, Rhenium, Osmium und Bismut und eine röntgenopake Verbindung aus diesen Elementen, insbesondere deren Komplexe, wenn sie in Salzform vorliegen, und Bariumsulfat, Bismuttrioxid, Bromin, Iodin, Iodid, ionische jodhaltige Verbindungen (beispielsweise Amidotrizoesäure, Handelsnamen: Gastrolux®, Gastrografin®, Peritrast®), nichtionische jodhaltige Verbindungen (beispielsweise Verbindungen mit den Handelsnamen Ultravist®, Isovist®, Xenetix®), gasförmiges Kohlenstoffdioxid (CO₂), Titanoxid und Zirkonoxid.

Die obige Aufgabe wird ferner durch ein System aus einem Katheter mit einem Ballon und einem Implantat gelöst, wobei das Implantat oben beschrieben ist und das Implantat auf dem Ballon des Katheters angeordnet ist. Ein solches System eignet sich gut zum Einbringen des Implantats mit den oben angegebenen Vorteilen zur Behandlung in einen Organismus.

Die obige Aufgabe wird auch durch ein Verfahren zur Herstellung eines Implantats mit den folgenden Schritten gelöst:
o Bereitstellen einer Grundstruktur mit einer Vielzahl von durchgehenden Öffnungen,
o Aufbringen einer Beschichtung, welche im dilatierten Zustand mindestens 20% der Querschnittsfläche, vorzugsweise mindestens 50% der Querschnittsfläche, mindestens eines Teils der Öffnungen eines vorgegebenen Abschnitts der Grundstruktur, vorzugsweise der Öffnungen des vorgegebenen Abschnitts mit der kleinsten Fläche, bedeckt und welche mindestens eine pharmazeutisch aktive Substanz freigibt.

Das angegebene erfindungsgemäße Verfahren ist ein einfaches Verfahren zur Herstellung eines erfindungsgemäßen Implantats.

Wie oben bereits diskutiert wurde, wird die Beschichtung vorzugsweise derart aufgebracht, dass höchstens jede zweite der nebeneinander liegenden, durchgehenden Öffnungen des vorgegebenen Abschnitts mit der mindestens eine pharmazeutisch aktive Substanz freigebenden Beschichtung bedeckt ist. Besonders bevorzugt wird die Beschichtung derart aufgebracht, dass höchstens 95% der Summe der Querschnittsflächen aller Öffnungen des vorgegebenen Abschnitts im dilatierten Zustand mit der mindestens eine pharmazeutisch aktive Substanz freigebenden Beschichtung bedeckt bzw. überspannt ist.

Es ist ferner von Vorteil, wenn die Beschichtung mittels Tauchen, Dippen, Sprühen, Pipettieren oder dergleichen aufgebracht wird, wobei die nicht zu beschichtenden Flächen mittels einer Maskierung und/oder einer Abdeckung geschützt werden können.

In einem bevorzugten Ausführungsbeispiel des erfindungsgemäßen Herstellungsverfahrens wird zuerst die Beschichtung aufgebracht, anschließend werden, vorzugsweise in einem gedehnten Zustand der Beschichtung, in die Beschichtung Poren eingebracht oder Poren aufgebrochen und während oder nach dem Einbringen oder Aufbrechen der Poren wird die mindestens eine pharmazeutisch aktive Substanz in die Poren eingebracht. Hierdurch wird erreicht, dass die pharmazeutisch aktive Substanz insbesondere erst nach dem Dilatieren des Implantats aus der Beschichtung freigesetzt wird. Ferner kann einfach gesteuert werden, in welche Richtung die Abgabe der mindestens einen pharmazeutisch aktiven Substanz erfolgt.

Die obige Aufgabenstellung wird auch gelöst durch ein Verfahren zur Herstellung eines Systems aus einem Katheter und einem Implantat, wobei zunächst das Implantat wie oben angegeben hergestellt wird und anschließend das Implantat auf dem Katheter, vorzugsweise auf dem Ballon des Katheters, befestigt, beispielsweise aufgecrimpt, wird. Dieses Herstellungsverfahren ist kostengünstig.

Die erfindungsgemäßen Verfahren bzw. das erfindungsgemäße Implantat und das erfindungsgemäße System werden nachfolgend in Beispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezüge.

Es zeigen schematisch:
- Figur 1: einen Abschnitt eines ersten Anführungsbeispiels eines erfindungsgenäßen Implantats in einer Ansicht von der Seite
- Figur 2: einen Abschnitt eines zweiten Ausführungsbeispiels eines erfindungsgemäßen Implantats in einer Ansicht von der Seite,
- Figur 3: einen Abschnitt eines dritten Ausführungsbeispiels eines erfindungsgemäßen Implantats in einer Ansicht von der Seite,
- Figur 4: einen Abschnitt eines vierten Ausführungsbeispiels eines erfindungsgemäßen Implantats, ebenfalls in einer Ansicht von der Seite,
- Figur 5: einen Ausschnitt einer Zelle eines erfindungsgemäßen Implantats mit Beschichtung in einer Ansicht von der Seite,
- Figur 6: einen Abschnitt eines fünften Ausführungsbeispiels eines erfindungsgemäßen Implantats in einer Ansicht von der Seite in einem nicht dilatierten Zustand (Figur 6a)) und in einem dilatierten Zustand (Figur 6b)) sowie
- Figur 7: einen Querschnitt durch eine Zelle des in Figur 5 dargestellten Ausführungsbeispiels eines erfindungsgemäßen Implantats.

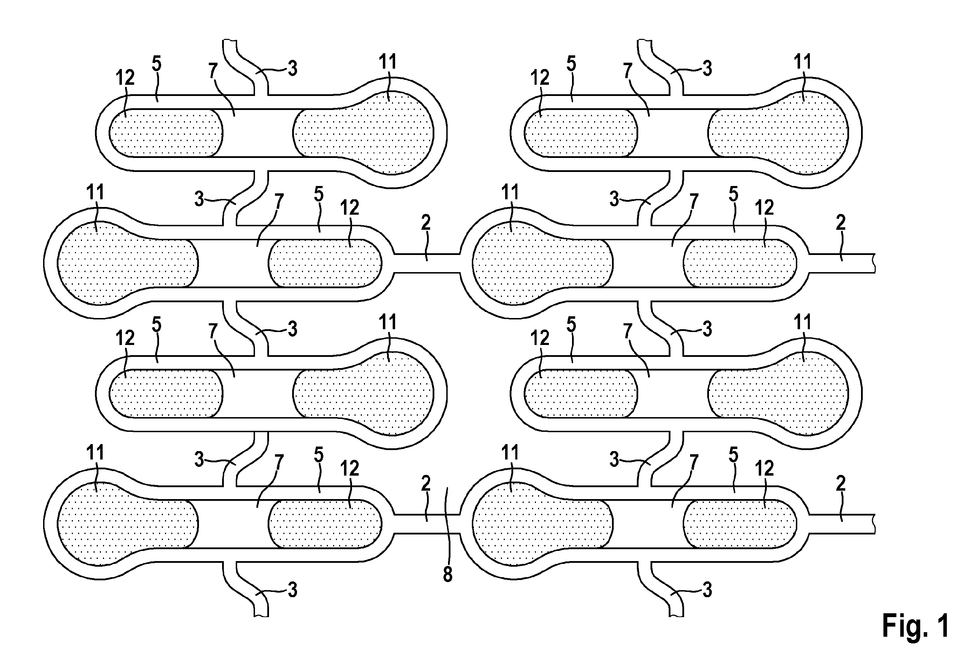

Der in Figur 1 gezeigte Abschnitt einer Grundstruktur eines hohlzylinderförmigen Stents besitzt gerade, in Längsrichtung verlaufende Stege 2 und S-förmig gebogene Stege 3, welche auf der Mantelfläche quer zur Längsrichtung verlaufen. Jeweils zwischen zwei in Längsrichtung verlaufenden Stegen 2 und zwei S-förmig gebogenen Stegen 3 sind zwei gebogene Stege 5 angeordnet, welche zusammen eine etwa schlüssellochförmige, durchgehende Öffnung 7 ausbilden. Die in Längsrichtung verlaufenden Stege 2 sind dabei nur in jeder zweiten, in Längsrichtung verlaufenden Reihe der durch die Stege 5 ausgebildeten Maschen angeordnet. Durch die beiden, die schlüssellochförmige Öffnung 7 ausbildenden Stege 5 wird eine Masche oder Zelle geformt. Zudem bilden jeweils sechs gebogene Stege 5, zwei gegenüber liegende, gerade Stege 2 und vier S-förmig gebogene Stege 3 zusammen eine Masche oder Zelle mit einer H-förmigen Öffnung 8 aus. Die Querschnittsfläche der schlüssellochförmigen Öffnung 7 ist kleiner als die Querschnittsfläche der H-förmigen Öffnung 8.

Die schlüssellochförmigen Öffnungen 7 sind zu etwa 70% ihrer Querschnittsfläche mit einer mindestens eine pharmazeutisch aktive Substanz enthaltenden Beschichtung bedeckt.

Die Beschichtung besteht aus einem Matrixmaterial, insbesondere aus Kautschuk, Latex, PLLA (Poly-L-Lactid), PLGA (Poly-L-Glycolide) oder aus PBMA (Poly-Butyl-Methacrylate) oder einem Formgedächtnis-Polymer. Die Beschichtung weist ferner eine oder mehrere pharmazeutisch aktiven Substanz(en), vorzugsweise Paclitaxel oder Sirolimus, auf. Die Freisetzung der pharmazeutisch aktiven Substanz(en) erfolgt durch Ausspülen mittels Körperflüssigkeit.

Die in Figur 1 gezeigte Beschichtung weist zwei Teile auf, einen ersten Teil 11 am Kopfende und einen zweiten Teil 12 am Fußende jeder schlüssellochförmigen Öffnung 7. Die H-förmigen Öffnungen 8 werden nicht von einer Beschichtung überspannt. Bei dieser Gestaltung der Beschichtung ist es möglich, durch das Design und die variable Befüllungsdichte die Wirkstoffmenge und -konzentration zu variieren.

Die Grundstruktur des Stents mit den Stegen 2, 3 und 5 besteht vorzugsweise aus der Metalllegierung CoCr, oder, in einem besonders bevorzugten Ausführungsbeispiel, aus einer biodegradierbaren Magnesiumlegierung.

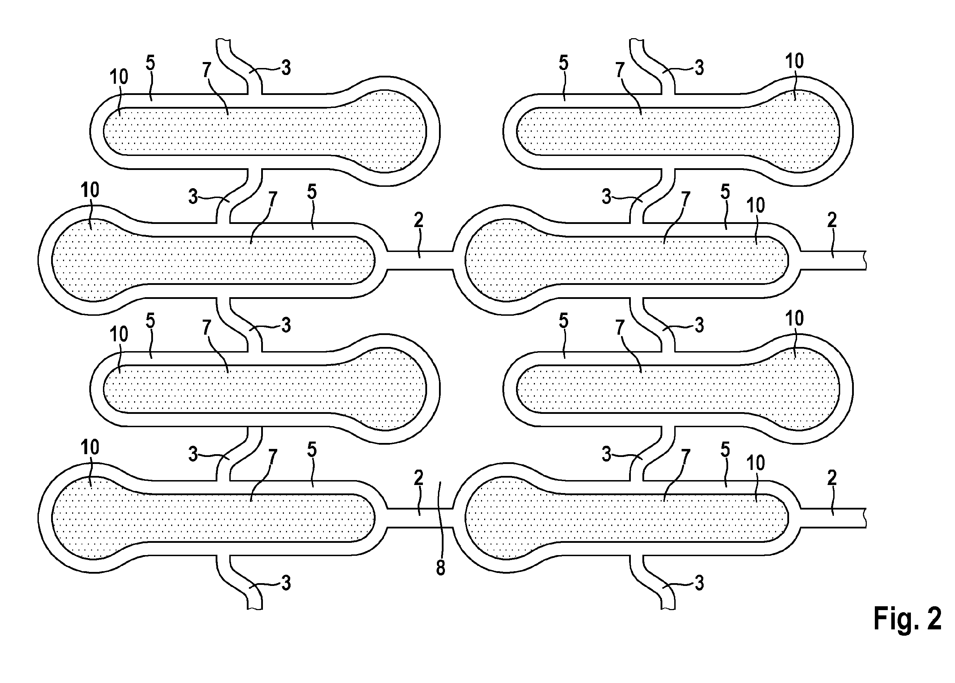

Bei dem in Fig. 2 dargestellten Ausführungsbeispiel sind 100% der Querschnittsfläche jeder schlüssellochförmigen Öffnung 7 mit einer Beschichtung 10 bedeckt. Neben den schlüssellochförmigen Öffnungen 7 enthält die Grundstruktur des Stents noch H-förmige Öffnungen, die jedoch analog zu dem in Fig. 1 gezeigten Ausführungsbeispiel nicht mit einer Beschichtung, welche die pharmazeutisch aktive Substanz enthält, abgedeckt sind.

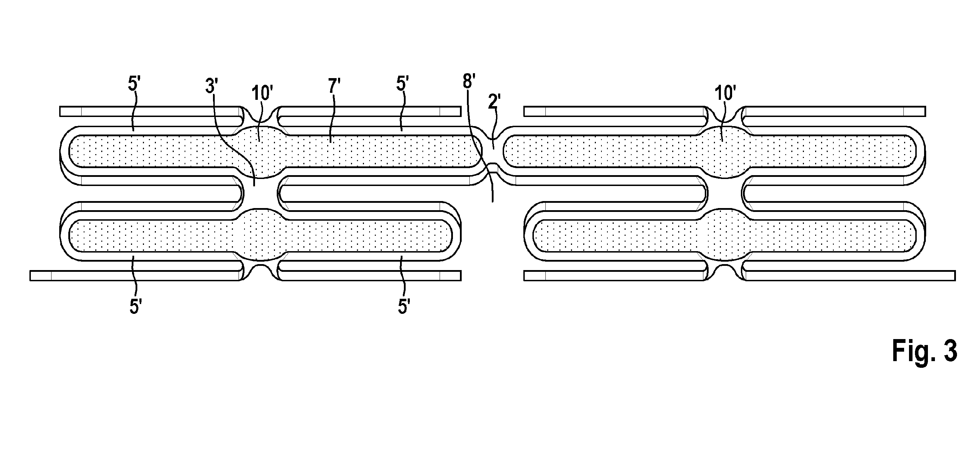

Auch in dem Ausführungsbeispiel, das in Fig. 3 gezeigt ist, ist die Querschnittsfläche, die durch die etwa ovalen Öffnungen 7' ausgebildet wird, zu 100% mit der Beschichtung 10' bedeckt. Die dazwischen liegenden, durch die Stege 2, 3' und 5' ausgebildeten etwa H-förmigen Öffnungen 8' sind jeweils nicht mit einer Beschichtung versehen.

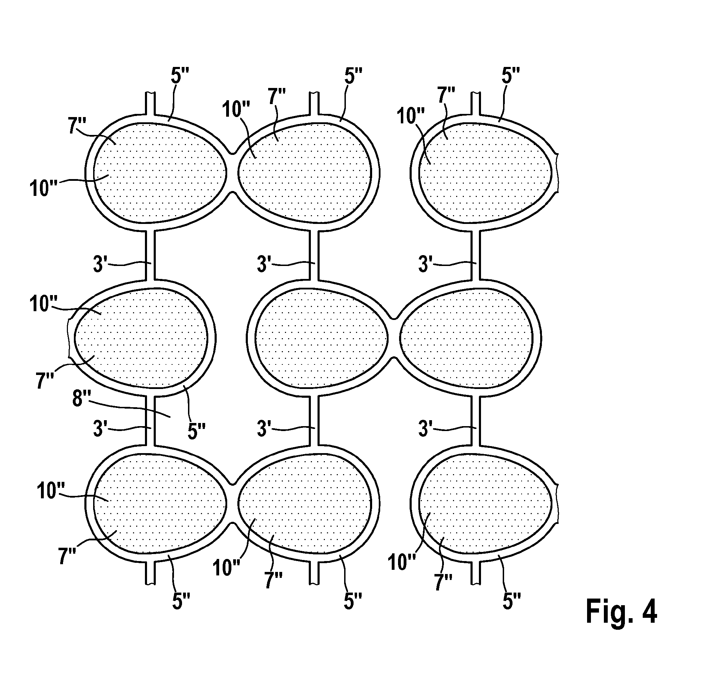

In Fig. 4 ist eine Grundstruktur eines erfindungsgemäßen Stents dargestellt, welche in etwa kreisförmige Maschen aufweist, die durch gebogene Stege 5" ausgebildet werden. Jede in etwa kreisförmige Querschnittsfläche der Öffnung 7" der Maschen ist vollständig mit der Beschichtung 10" bedeckt.

Die etwa kreisförmigen Maschen werden durch quer zur Längsrichtung verlaufende Stege 3' miteinander verbunden. In Richtung der Längsrichtung des Stents sind in diesem Ausführungsbeispiel keine separaten Stege angeordnet. Die durch die Stege 3' und die Stege 5" ausgebildeten, H-förmigen Maschen 8" tragen keine Beschichtung.

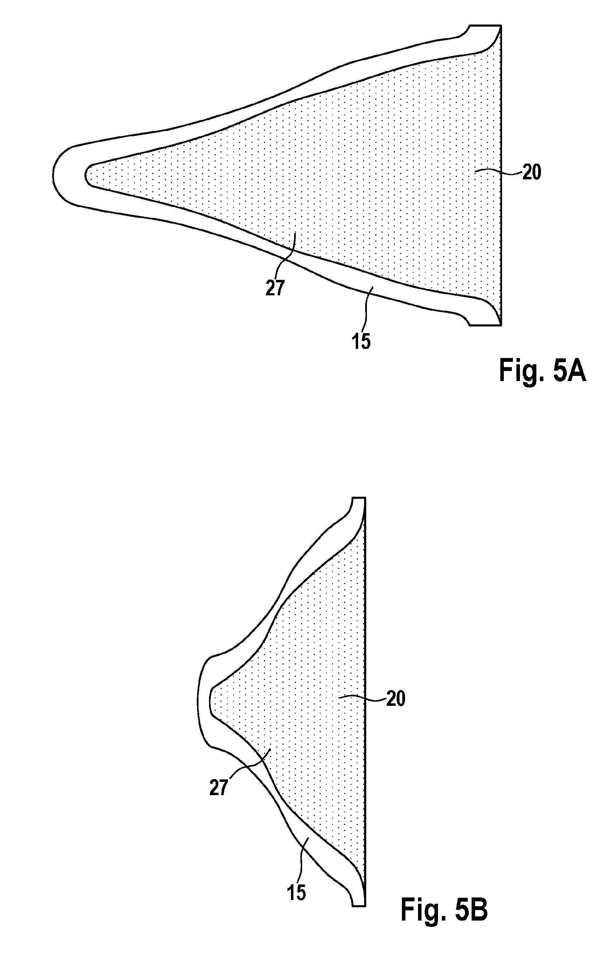

Fig. 5 zeigt den Abschnitt eines Stegs 15, welcher eine Masche oder Zelle der Grundstruktur eines erfindungsgemäßen Implantats ausbildet, in einer Ansicht aus luminaler Richtung. Dieser Steg ist mit einer mindestens eine pharmazeutisch aktive Substanz enthaltenden Beschichtung 20 bedeckt, welche jeweils eine durch den Steg 15 ausgebildete durchgehende Öffnung 27 überspannt. In den Figuren 5a) und 5b) sind jeweils Abschnitte der gleichen Maschen dargestellt, wobei der in Fig. 5a) dargestellte Steg 15 mit Beschichtung 20 zu einem nicht dilatierten Implantat gehört, während der Steg 15, welche in Fig. 5b) gezeigt ist, mit dem entsprechenden Implantat dilatiert wurde. Die Beschichtung 20 ist elastisch, so dass sie beide Zustände einnehmen kann, ohne zu reißen.

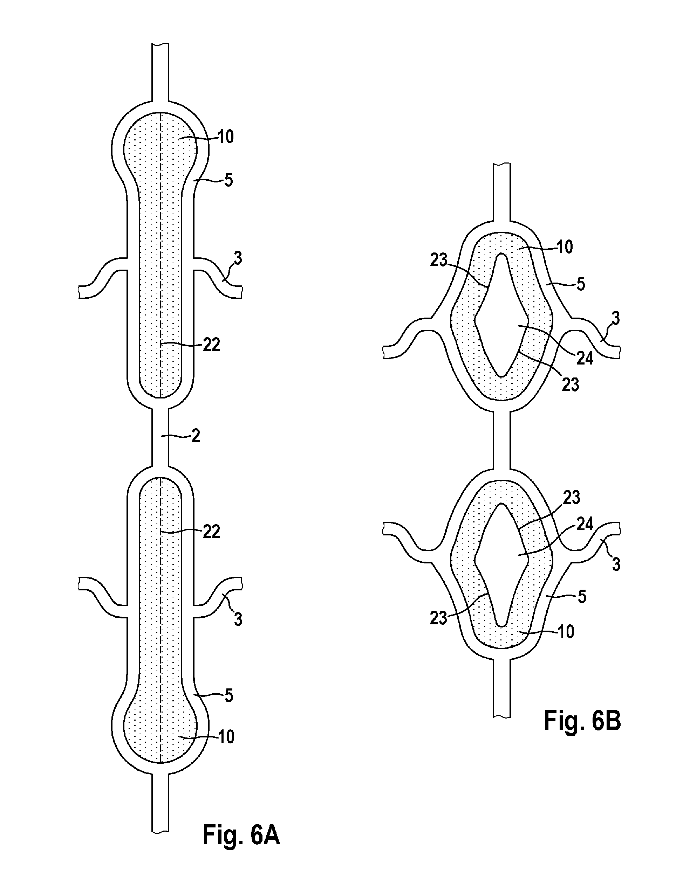

In Fig. 6 ist ein weiteres Ausführungsbeispiel eines erfindungsgemäßen Implantats dargestellt, das im Wesentlichen dem anhand Fig. 2 dargestellten Ausführungsbeispiel entspricht. Im Unterschied zu dem zweiten Ausführungsbeispiel sind die schlüssellochförmigen Öffnungen gegensinnig angeordnet. Ferner weist die Beschichtung 10 etwa entlang einer Mittellinie eine gestrichelt gezeichnete Perforationslinie 22 auf, die im nicht dilatierten Zustand (siehe Fig. 6a)) jedoch noch geschlossen ist und eine Sollbruchlinie darstellt. Entlang der Perforationslinie 22 sind voneinander beabstandete Löcher in der Beschichtung 10 angeordnet und/oder weist die Beschichtung 10 eine geringfügig kleinere Schichtdicke auf. Während der Dilatation des Implantats nach der Anordnung an der zu behandelnden Stelle im Körper ist die Dehnung der Beschichtung 10 so stark, dass die Perforationslinie 22 aufreißt und eine Öffnung 24 ausbildet. Die Öffnung 24 wird durch die Kanten 23 umschlossen, welche entlang der zuvor, d.h. im nicht dilatierten Zustand, vorliegenden Perforationslinie 22 verlaufen. Das Aufreißen der Beschichtung 10 entlang einer vorgegebenen Perforationslinie 22 während der Dilatation hat den Vorteil, dass einerseits eine Öffnung 24 in der Beschichtung 10 an einer definierten Stelle entsteht. Andererseits wird durch das Aufreißen der Beschichtung 10 die Freisetzung der in der Beschichtung 10 enthaltenen pharmazeutisch aktiven Substanz(en) begünstigt.

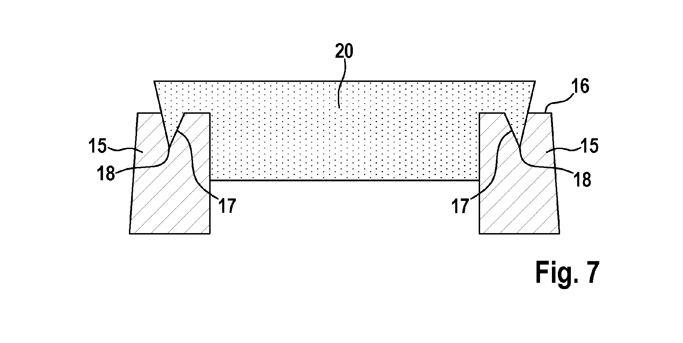

Fig. 7 zeigt das in Fig. 5 bereits dargestellte Ausführungsbeispiel in einem Querschnitt. Die Verankerung der Beschichtung 20 erfolgt mittels eingelaserter Nuten (Schlitze) 17, welche auf der abluminalen Seite 16 des jeweiligen Stegs 15 angeordnet sind. Hierbei wird die Beschichtung 20 mittels eines Formschlusses und/oder mittels eines nicht dargestellten Klebstoffs (Stoffschluss) in den Nuten 17 befestigt. Alternativ kann eine stoffschlüssige Verbindung zwischen Beschichtung 20 und Steg 15 auch ohne Nut erfolgen. In Fig. 5 ist der Verlauf der Bodenlinie 18 der im Querschnitt dreieckförmigen Nut 17 als strichpunktierte Linie veranschaulicht.

Die in den Figuren 1 bis 6 dargestellte Beschichtung 10, 10', 10", 11, 12 und 20 dient als vorzugsweise hochelastischer Träger einer oder mehrerer pharmazeutisch aktiver Substanzen. Diese Substanzen können beispielsweise während der Herstellung der Beschichtungslösung bereits durch Tauchen oder Eindiffundieren in die Lösung und somit in das zukünftige Polymer eingebracht werden. Ferner können zusätzlich Röntgenmarker bzw. röntgenopake Materialien in der Beschichtung vorgesehen sein. Diese können beispielsweise mittels eingeschlossener Partikel, Tauchen, Eindiffundieren, Besputtern, elektrochemische oder statische Abscheideprozesse, evtl. mit nachträglicher Wärmebehandlung zum Einbringen bzw. Fixieren, in die Schicht bzw. das Schichtmaterial eingebettet werden.

Die Beschichtung wird mittels Dippen, Sprühen, Pipettieren oder dergleichen aufgebracht, wobei die nicht zu beschichtenden Flächen mittels einer Maskierung, einer hydrophoben Beschichtung und/oder einer Abdeckung geschützt werden.

In einem Ausführungsbeispiel werden alle nicht zu beschichteten Flächen oder Aussparungen (Freiflächen) mit einer Abdeckung und/oder einer hydrophoben Schutzschicht versehen. Danach wird das Implantat durch Tauchen oder Dippen zur Herstellung der mindestens eine pharmazeutisch aktive Substanz freigebenden Beschichtung beschichtet. Wenn die Beschichtung in diesem Schritt von allen Seiten erfolgt, wird insbesondere die luminale Seite des Stents abgedeckt. Hierbei haftet das Beschichtungsmaterial nicht an den Flächen des Stents, die mit der Abdeckung bzw. Schutzschicht versehen wurden. Im Nachgang werden dann die Abdeckungen bzw. Schutzschichten durch Erwärmen, Abziehen oder Ätzen entfernt. Hierbei kann die hydrophobe Schutzschicht auch auf den Stegen verbleiben, wenn sie biokompatibel ausgeführt ist.

Prinzipiell wird erreicht, dass die Beschichtung sich nicht nur auf den Stegen anlagert, sondern gezielt bestimmte Öffnungen bzw. einen bestimmten Bereich bestimmter Öffnungen überspannt, indem die Beschichtung durch gezieltes Pipettieren hergestellt wird, wenn nicht alle Öffnungen beschichtet werden sollen. So wird gewährleistet, dass, wie in Fig. 7 dargestellt, alle Haltebereiche genutzt werden. Nach dem Aushärten der Matrix können gezielt Fehlstellen in bestimmten Bereichen der Beschichtung eingebracht werden. Geeignete Methoden wären beispielsweise Laserschneiden, -punktieren oder Einschneiden. In einem weiteren Ausführungsbeispiel können auch sogenannte Sollbruchstellen in die Beschichtung eingestanzt oder eingewalzt werden. Beim Dilatieren reißt die Beschichtung dann genau an dieser Stelle ein.

In der Beschichtung können auch Formgedächtnis-Polymere verwendet werden. Als erstes Element oder Grundbaustein für ein Formgedächtnis-Polymer können alle Materialien verwendet werden, aus denen Polymere hergestellt werden können. Es ist ferner bevorzugt, wenn diese Materialien biokompatibel sind. Das zweite Element zur Herstellung eines Formgedächtnis-Materials bewirkt die Programmierung, mit der der Kunststoff in seine zweite, temporäre Form gebracht wird. Nach der Herstellung des Polymers für ein bestimmtes Einsatzgebiet wird dieser klassisch verarbeitet. Hierbei wird das Material einmal aufgeschmolzen und abgekühlt. So erhält der Kunststoff seine erste, permanente Form. Anschließend wird das Material erneut erhitzt, und zwar über seine so genannte Schalttemperatur hinaus. Diese Schalttemperatur ergibt sich aus den spezifischen Eigenschaften des Materials und basiert auf einem Phasenübergang der Polymerketten, welcher deutlich unterhalb der ursprünglichen Verarbeitungstemperatur liegt. Der Kunststoff wird dabei zum zweiten Mal verformt, wieder unter die Schalttemperatur abgekühlt und hierdurch "programmiert", d.h. in seiner zweiten temporären Form fixiert. In dieser Form wird die Beschichtung dann an ihren Einsatzort gebracht. Dort erhält das Material seinen spezifischen thermischen Stimulus, beispielsweise durch die Körpertemperatur des behandelten

Organismus oder über eine warme Flüssigkeit, die über eine Sonde zu dem Polymer gebracht wird. Durch diesen Stimulus nimmt das Polymer am Behandlungsort wieder seine erste, permanente Form an. Die genannten Formgedächtnis-Polymere (FGP) sind somit besonders gut dazu geeignet, eine pharmazeutisch aktive Substanz in verschiedenster Form aufzunehmen und diese am Behandlungsort auch wieder abzugeben, da in diese Polymere Poren vorgesehen ("einprogrammiert") werden können, die erst nach der Dilatation am Implantationsort wieder aufgehen und den Wirkstoff freigeben können. Das Einbringen der pharmazeutisch aktiven Substanz erfolgt während oder nach der "Programmierung". Beispiele für Polymere mit Formgedächtniseffekt sind Multiblock-Copolymere oder Standardpolymere (PET, PS, PUR, PE, PES, PTFE). Die Einprogrammierung erfolgt beispielsweise durch kurzes Erwärmen des Formgedächtnis-Polymers auf die Formgedächtnistemperatur, wobei sich das Polymer aufdehnt. In dieser Phase können Poren aufbrechen oder mittels Stanzen oder Ätzen eingebracht werden. Jetzt kann der Wirkstoff in die Poren gefüllt werden, z.B. durch Diffusion.

Die maximale Schichtdicke der Beschichtung beträgt vorzugsweise 100 µm. Bei einer Schichtdicke von mehr als 100 µm ist die erforderliche Elastizität der Beschichtung nicht gewährleistet.

Die erfindungsgemäßen Implantate zeichnen sich dadurch aus, dass eine große Fläche für die Freisetzung einer pharmazeutisch aktiven Substanz geschaffen wird. Hierdurch ist eine gleichmäßigere Verteilung des Wirkstoffs in dem behandelten Bereich des Organs möglich. Die Dosierung der Wirkstoffe kann somit erhöht werden. Ferner ist auch die Verteilung der Beschichtung an verschiedenen Abschnitten des Implantats unterschiedlich gestaltbar, beispielsweise unterschiedlich am proximalen oder am distalen Ende des Implantats.

### Bezugszeichenliste

- 2: Steg
- 3, 3': Steg
- 5, 5', 5": Steg
- 7: schlüssellochförmige Öffnung
- 7': ovale Öffnung
- 7": kreisförmige Öffnung
- 8, 8', 8": H-förmige Öffnung
- 10, 10', 10": Beschichtung
- 11: Beschichtung
- 12: Beschichtung
- 15: Steg
- 16: abluminale Seite
- 17: Nut
- 18: Bodenlinie
- 20: Beschichtung
- 22: Perforationslinie
- 23: Kante
- 24: Öffnung
- 27: Öffnung

## Patentansprüche

1. Implantat mit einer vorzugsweise hohlzylinderförmigen Grundstruktur mit einer Vielzahl von durchgehenden Öffnungen (7, 7', 7", 8, 8') und mit einer Beschichtung, welche mindestens eine pharmazeutisch aktive Substanz freigibt, **dadurch gekennzeichnet, dass** mindestens 20% der Querschnittsfläche, vorzugsweise mindestens 50% der Querschnittsfläche, mindestens eines Teils der Öffnungen (7, 7', 7", 27) eines vorgegebenen Abschnitts der Grundstruktur, vorzugsweise der Öffnungen des vorgegebenen Abschnitts mit der kleinsten Querschnittsfläche, im dilatierten Zustand mit der mindestens eine pharmazeutisch aktive Substanz freigebenden Beschichtung (10, 10', 10", 11, 12, 20) bedeckt ist.

2. Implantat nach Anspruch 1, **dadurch gekennzeichnet, dass** höchstens jede zweite der nebeneinander liegenden, durchgehenden Öffnungen des vorgegebenen Abschnitts mit der mindestens eine pharmazeutisch aktive Substanz freigebenden Beschichtung (10, 10', 10", 11, 12, 20) bedeckt ist.

3. Implantat nach einem der vorhergehenden Ansprüche, **dadurch gekennzeichnet, dass** jede durchgehende Öffnung in einer abgeschlossenen Zelle der Grundstruktur enthalten ist, wobei die Zelle durch zwei oder mehr, gegebenenfalls geknickte und/oder gebogene Stege (2, 3, 3', 5, 5', 15) ausgebildet wird.

4. Implantat nach einem der vorhergehenden Ansprüche, **dadurch gekennzeichnet, dass** die pharmazeutisch aktive Substanz aus der Beschichtung (10, 10', 10", 11, 12, 20) mindestens überwiegend in abluminale Richtung abgegeben wird.

5. Implantat nach einem der vorhergehenden Ansprüche, **dadurch gekennzeichnet, dass** die Beschichtung mehrere Lagen aufweist, wobei die erste, am weitesten in luminaler Richtung angeordnete Lage vorzugsweise als Diffusionsbarriere für die mindestens eine pharmazeutisch aktive Substanz ausgebildet ist.

6. Implantat nach einem der vorhergehenden Ansprüche, **dadurch gekennzeichnet, dass** das Material der Beschichtung (10, 10', 10", 11, 12, 20) einen E-Modul im Bereich von 0,01 kN/mm² bis 4 kN/mm², vorzugsweise einen E-Modul im Bereich von 0,01 kN/mm² bis 2 kN/mm², aufweist.

7. Implantat nach einem der vorhergehenden Ansprüche, **dadurch gekennzeichnet, dass** die Beschichtung (10, 10', 10", 11, 12, 20) mindestens ein Polymer der Gruppe enthaltend die Polymere der Klasse der Elastomere, insbesondere Kautschuk und Latex, weiter enthaltend PET, PS, PA, PUR, PE, PES, PTFE, PLA, PLLA, PLGA, PGA, PBMA, PEVA, Formgedächtnis-Polymere (insbesondere Multiblock-Copolymere oder Standardpolymere wie PET, PS, PUR, PE, PES, PTFE) sowie deren Blends sowie Co- und Blockpolymere, aufweist.

8. Implantat nach einem der vorhergehenden Ansprüche, **dadurch gekennzeichnet, dass** die Beschichtung (10', 10", 11, 12, 20) mindestens einen Röntgenmarker und/oder ein Material zur Verbesserung der MRI-Sichtbarkeit aufweist.

9. Implantat nach einem der vorhergehenden Ansprüche, **dadurch gekennzeichnet, dass** die Grundstruktur, vorzugsweise ein Teil der Stege (15), vorzugsweise auf der abluminalen Seite eine Nut (17) zur Befestigung der Beschichtung (20) aufweist.

10. System aus einem Katheter mit einem Ballon und einem Implantat nach einem der vorhergehenden Ansprüche, **dadurch gekennzeichnet, dass** das Implantat auf dem Ballon des Katheters angeordnet ist.

11. Verfahren zur Herstellung eines Implantats mit einer vorzugsweise hohlzylinderförmigen Grundstruktur mit den folgenden Schritten:
- Bereitstellen einer Grundstruktur mit einer Vielzahl von durchgehenden Öffnungen (7, 7', 7", 8, 8'),
- Aufbringen einer Beschichtung (10, 10', 10", 11, 12, 20), welche im dilatierten Zustand mindestens 20% der Querschnittsfläche, vorzugsweise mindestens 50% der Querschnittsfläche, mindestens eines Teils der Öffnungen (7, 7', 7", 27) eines vorgegebenen Abschnitts der Grundstruktur, vorzugsweise der Öffnungen des vorgegebenen Abschnitts mit der kleinsten Querschnittsfläche, bedeckt und mindestens eine pharmazeutisch aktive Substanz freigibt.

12. Verfahren nach Anspruch 11, **dadurch gekennzeichnet, dass** die Beschichtung (10, 10', 10", 11, 12, 20) derart aufgebracht wird, dass höchstens jede zweite der nebeneinander liegenden, durchgehenden Öffnungen des vorgegebenen Abschnitts mit der Beschichtung bedeckt ist.

13. Verfahren nach einem der Ansprüche 11 bis 12, **dadurch gekennzeichnet, dass** die Beschichtung (10, 10', 10", 11, 12, 20) mittels Tauchen, Dippen oder Sprühen aufgebracht wird, wobei die nicht zu beschichtenden Flächen oder Flächenbereiche mittels einer Maskierung und/oder einer Abdeckung geschützt werden.

14. Verfahren nach einem der Ansprüche 11 bis 13, **dadurch gekennzeichnet, dass** zuerst die Beschichtung aufgebracht wird, anschließend, vorzugsweise in einem gedehnten Zustand der Beschichtung, in die Beschichtung Poren eingebracht oder Poren aufgebrochen werden und während oder nach dem Einbringen der Poren oder Aufbrechen der Poren die mindestens eine pharmazeutisch aktive Substanz in die Poren eingebracht wird.

15. Verfahren zur Herstellung eines Systems aus einem Katheter und einem Implantat, wobei zunächst das Implantat nach einem der Ansprüche 11 bis 14 hergestellt wird und anschließend das Implantat auf dem Katheter, vorzugsweise auf einem Ballon des Katheters, befestigt wird.
